(19) Europäisches Patentamt
European Patent Office
Office européen des brevets

(11) Publication number: **0 040 435**
**B1**

(12) # EUROPEAN PATENT SPECIFICATION

(45) Date of publication of the patent specification: **20.07.83**

(51) Int. Cl.³: **C 07 C 103/52,** A 61 K 37/02, A 61 K 37/64

(21) Application number: **81103882.7**

(22) Date of filing: **20.05.81**

(54) **Peptide derivatives and process for the preparation thereof.**

(30) Priority: **20.05.80 JP 65973/80**
**20.05.80 JP 65974/80**

(43) Date of publication of application:
**25.11.81 Bulletin 81/47**

(45) Publication of the grant of the patent:
**20.07.83 Bulletin 83/29**

(84) Designated Contracting States:
**DE FR GB NL**

(56) References cited:
**GB-A-1 486 276**
**US-A-3 907 764**
**US-A-3 975 366**
**US-A-4 070 458**

(73) Proprietor: **Eisai Co., Ltd., 6-10, Koishikawa 4-chome Bunkyo-ku, Tokyo 112 (JP)**

(72) Inventor: **Kuwana, Noriaki, 5-3, Tatsuga-ike, Inuyama-shi Aichi Prefecture (JP)**
Inventor: **Hasegawa, Yoshikazu, 6-74, Matsuga-oka, Kagamigahara-shi Gifu Prefecture (JP)**
Inventor: **Nozu, Yukio, 1225-1, Matsukura-machi Kawashima-cho, Hashima-gun Gifu Prefecture (JP)**

(74) Representative: **Hansen, Bernd, Dr.rer.nat. et al, Hoffmann . Eitle & Partner Patentanwälte Arabellastrasse 4, D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

# Peptide Derivatives and Process for the Preparation Thereof

This invention relates to peptide derivatives and process for the preparation thereof.

The compounds of the present invention are represented by the general formula

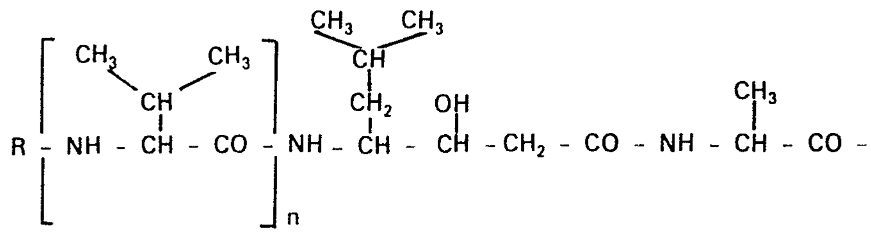

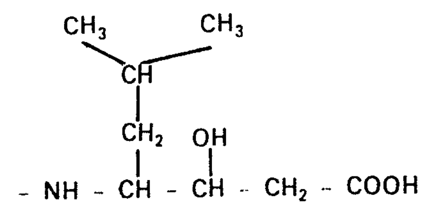

(I)

wherein n is an integer of 1 or 2, and if n is 1, R stands for a radical selected from the group consisting of carboxypropanoyl, nicotinoyl and benzyloxycarbonyl-leucyl-leucyl, and, if n is 2, R stands for a radical selected from the group consisting of carboxypropanoyl, 2-carboxybenzoyl, nicotinoyl and benzyloxycarbonyl-leucyl-leucyl.

US-A-4,070,458 describes structurally similar compounds which are reported to be effective on gastric ulcers due to their anti-pepsin activity. The above-identified reference does not describe any inhibitory effect on the endopeptidase renin.

GB-A-1,486,276 describes a process for the production of N-acylpentapeptides by culture of a strain of Streptomyces. The N-acylpentapeptides are useful because of their antipepsin activity. Also US-A-3,975,366 describes a process for producing new pepstatins, which possess anti-pepsin activity. Also US-A-3,907,764 describes protease inhibitors with a strong inhibitory action against pepsin and other acid proteases. None of the above-identified references, however, disclose compounds showing an anti-renin activity besides the inhibitory effect on pepsin.

The peptide derivatives of the present invention possess strong pepsin-inhibitory activities and also an acid portease-inhibitory activity such as anti-renin activity and therefore are useful for therapeutic treatment of diseases caused by hyper-secretion of pepsin and for the prophylaxis and treatment of hypertension.

According to the present invention, the compounds having the general formula (I) in which n is as defined above and R is carboxy-propanoyl, 2-carboxy-benzoyl or nicotinoyl, can be prepared by the reaction of compound having the formula:

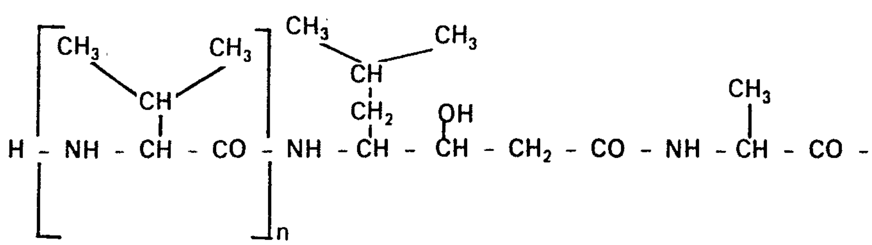

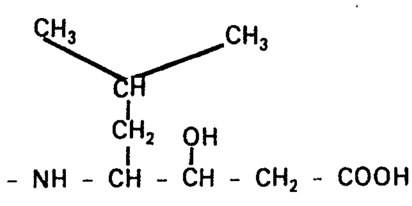

(II)

wherein n is as defined above, with an organic carboxylic acid selected from the group consisting of succinic, phthalic and nicotinic acids or reactive derivatives thereof. Examples of the reactive derivatives of the above organic acids include acid anhydrides, acid halides and the like.

In case of using the organic acid in free form, the above reaction is advantageously effected in the presence of a condensing agent known for the amide forming reaction. The reaction may be carried out according to the conventional procedure used in the amide forming reaction well known in the art.

A compound of formula (I) in which n is an integer of 1 or 2 and R stands for benzyloxycarbonyl-leucyl-leucyl, may be obtained first by reacting a compound of the formula (II) with a N-protecting leucine ester, followed by splitting off the protecting group of the product. The resulting leucyl-derivative is then reacted with carboxy-benzoyl-leucine ester to obtain the compound of formula (I) wherein n is as defined above and R stands for benzyloxycarbonyl-leucyl-leucyl.

The kind of the N-protecting groups and alcohol moieties of the reactive leucine ester to be used for the reaction and the condition of the reaction may suitably be chosen upon the knowledges for the synthesis of peptides known by those skilled in the art.

The starting material of the formula (II) used in the present invention is disclosed in the specification of Japanese Patent Application Laid-open No. 67791/76. Thus, they may be obtaines by the action of, strain of a bacterium belonging to Bacillus sp., e.g., Bacillus pumilus, Kawaguchi (Ferm-p-2677) EF49-210, to the known N-acylpentapeptides, such as Pepsidines A, B and C, and Pepstatines, which are obtained by cultivation of various streptomyces [See; Agr. Biol. Chem. Japan, 40(3) 451 (1976)]. In above patent specification, the compound of the formula (II) in which n is 1 or 2, is designated as DAV-pepsidine and DA-pepsidine, respectively.

The following Examples will specifically illustrate embodiment of the present invention, however, should not be recognized as to limit the scopes of this invention.

Example 1

Preparation of N-3-carboxypropanoyl-DAV-pepsidine (formula I; R = 3-carboxypropanoyl, n = 1)

Three-fold molar amount (530 mg) of succinic anhydride was added to an aqueous solution (36 ml) of DAV-pepsidine (formula II; n = 1) (0.9 g). The mixture obtained was stirred for three hours at a room temperature while keeping pH 8.5 with addition of 5N aqueous sodium hydroxide solution.

When the reaction was over, the reaction solution was extracted three times with an equal volume of n-butanol. The combined extracts were concentrated to dryness. The residue was dissolved in ethanol. With addition of ethyl ether to the ethanol solution, an amorphous powder (980 mg) separated out was recovered by filtration. It was purified by column chromatography on silica gel, using as eluting solvent a mixture consisting of n-butanol:acetic acid:water:n-butyl acetate (4:1:1:4 by volume), the solvent being hereinafter designated as "BAWB-Solvent". N-3-carboxypropanoyl-DAV-pepsidine (830 mg; 77.0%) was thus obtained in a form of white powder melting at 153°–158°C. with decomposition.

Elementary analysis: as $C_{28}H_{48}N_4O_8 \cdot \frac{1}{2}H_2O$

| | | | |
|---|---|---|---|
| Calculated (%): | C 54.98 | H 8.40 | N 9.16 |
| Found (%): | C 54.98 | H 8.59 | N 8.77 |

TLC: Rf = 0.45 on Kiesel Gel $60G_{254}$ Plate (BAWB-Solvent)

Example 2

Preparation of N-nicotinoyl-DAV-pepsidine (formula I; R = nicotinoyl, n = 1)

To an aqueous solution (90 ml) which contained 0.9 g of DAB-pepsidine was added 3.5-fold molar amount (1110 mg) of nicotinoyl chloride hydrochloride. Further procedures were then followed with those mentioned in the above Example 1, with exception that a mixture consisting of n-butanol, acetic acid and water (3:1:1 by volume) was used as the eluting solvent in the chromatography.

There was thus obtained N-nicotinoyl-DAV-pepsidine (597 mg; 54.9%) in a white powder which had the melting point of 179°–187°C. with decomposition.

Elementary analysis: $C_{30}H_{49}N_4O_8$

| | | | |
|---|---|---|---|
| Calculated (%): | C 59.29 | H 8.13 | N 11.52 |
| Found (%): | C 59.42 | H 8.14 | N 11.33 |

TLC: Rf = 0.45 on Kiesel $Gel_{254}$ Plate (BAWB-Solvent)

Example 3

Preparation of benzyloxycarbonyl-leucyl-leucyl-DAV-pepsidine

(formula I; R = benzyloxycarbonyl-leucyl-leucyl, n = 1)

DAV-pepsidine (1.3 g) was dissolved in dimethyl formamide (20 ml). To the solution was added an equimolar amount (1 g) of benzyloxycarbonyl-leucine p-nitrophenyl ester, and the mixture was stirred for 18 hours at room temperature. When the reaction was completed, the reaction mixture was concentrated to dryness. The residue obtained was dissolved in acetone (300 ml) under warming. The acetone solution was then concentrated up to 100 ml and kept in a refrigerator overnight.

There was obtained benzyloxycarbonyl-leucyl-DAV pepsidine (821 mg; 43%) in a white powder.

The product (791 mg) obtained above was dissolved in methanol (50 ml). To the resulting solution was added 0.5% palladium on calcium carbonate (300 mg), and the mixture was subjected to catalytic reduction in accordance with a conventional manner. After filtration, the resulting solution was evaporated to dryness. The residue was dissolved in ethanol and the solution thus obtained was cooled to obtain leucyl-DAV-pepsidine in a white powder (535 mg; 81%).

Leucyl-DAV-pepsidine (535 mg) obtained above was dissolved in dimethylformamide (28 ml). To the solution was added 1.2-fold molar amount (403 mg) of benzyloxycarbonyl leucine p-nitrophenyl ester and the mixture was stirred for 24 hours at room temperature. The reaction mixture was then concentrated to obtain an oily residue, which was triturated with ethyl ether. A white powder (760 mg) formed was filtrated off and recrystallized from acetone. There was thus obtained benzyloxycarbonyl-leucyl-leucyl-DAV-pepsidine (568 mg; 76%) in a white powder, which had the melting point of 212°–214°C. with decomposition.

Elementary analysis: as $C_{44}H_{74}N_6O_{11} \cdot ½H_2O$

| | | | |
|---|---|---|---|
| Calculated (%): | C 60.60 | H 8.67 | N 9.64 |
| Found (%): | C 60.61 | H 8.72 | N 9.72 |

TLC: Rf = 0.73 on Kiesel Gel $60G_{254}$ Plate (BAWB-Solvent)

Example 4

Preparation of N-3-carboxy propanoyl-DA-pepsidine (formula I; R = 3-carboxy-propanoyl, n = 2)

The value of 1.2-fold molar amount (179 mg) of succinic anhydride was added to an aqueous solution (36 ml) of DA-pepsidine (formula II, n = 2) (0.9 g). The mixture was stirred for two hours at room temperature while the pH of said mixture was maintained to 8.5 with addition of 5N aqueous sodium hydroxide solution. After the completion of the reaction, the reaction mixture was concentrated to about 20 ml and acidified to pH 3–4 with addition of a 10% aqueous citric acid solution under ice-cooling and allowed to stand under ice-cooling for one hour. The resulting precipitate was recovered by filtration and washed with ethyl ether.

There was thus obtained N-3-carboxypropanoyl-DA-pepsidine (825 mg; 78.6%) in the form of white powder, which had the melting point of 205°–210°C with decomposion.

Elementary analysis: $C_{33}H_{59}N_5O_{11} \cdot ½H_2O$

| | | | |
|---|---|---|---|
| Calculated (%): | C 55.76 | H 8.51 | N 9.85 |
| Found (%): | C 55.69 | H 8.39 | N 9.55 |

TLC: Rf = 0.47 on Kiesel Gel $60G_{254}$ Plate (BAWB-Solvent)

Example 5

Preparation of N-2-carboxybenzoyl-DA-pepsidine (formula I; R = carboxybenzoyl, n = 2)

To an aqueous solution (32 ml) of DA-pepsidine (0.8 g) was added 1.1-fold molar amount (197 mg) of phthalic anhydride.

Further procedures were followed in accordance with those mentioned in the preceding Example 5. There was thus obtained N-2-carboxybenzoyl-DA-pepsidine (734 mg; 73.6%) in a form of white powder, the melting point of which was 165° -174°C.

Elementary analysis: $C_{37}H_{59}N_5O_{11} \cdot H_2O$

| | | | |
|---|---|---|---|
| Calculated (%): | C 57.87 | H 8.00 | N 9.12 |
| Found (%): | C 57.52 | H 7.84 | N 9.00 |

TLC: Rf = 0.51 on Kiesel Gel $60G_{254}$ Plate (BAWB-Solvent)

Example 6

Preparation of N-nicotinoyl-DA-pepsidine (formula I; R = nicotinoyl, n = 2)

To an aqueous solution (30 ml), which contained 0.9 g of DA-pepsidine, was added threefold molar amount (786 mg) of nicotinoyl chloride hydrochloride. The mixture was stirred for three hours at room temperature while the pH of mixture was maintained at 8.5 with addition of 5N aqueous sodium hydroxide solution.

Thereafter, additonal nicotinoyl chloride hydrochloride (158 mg) was again added to the reaction mixture. The pH of the reaction mixture was adjusted to 4 and allowed to stand for one hour under ice-cooling. The precipitate separated out was recovered by filtration and washed with ethyl ether. The product was then purified by column chromatography on silica gel using as eluting solvent a mixture consisting of n-butanol, acetic acid and water (3:1:1 by volume).

There was thus obtained N-nicotinoyl-DA-pepsidine (522 mg; 49.3%) in a form of white powder, the melting point of which was 221°–226°C with decomposition.

Elementary analysis: $C_{35}H_{58}N_6O_9 \cdot ½H_2O$

| | | | |
|---|---|---|---|
| Calculated (%): | C 58.72 | H 8.31 | N 11.74 |
| Found (%): | C 58.67 | H 8.35 | N 11.67 |

TLC: Rf = 0.47 on Kiesel Gel $60G_{254}$ Plate (BAWB-Solvent)

## Claims

1. Peptide derivatives represented by the general formula:

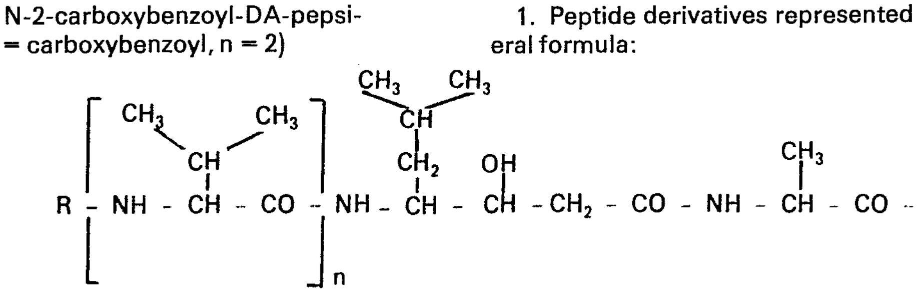

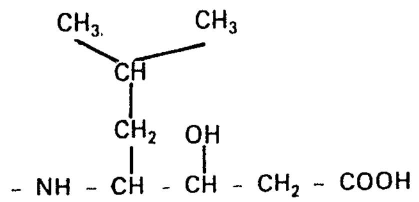

(I)

wherein n is an integer of 1 or 2, and if n is 1, R stands for a radical selected from the group consisting of carboxypropanoyl, nicotinoyl and benzyloxycarbonyl-leucyl-leucyl, and, if n is 2, R stands for a radical selected from the group consisting of carboxypropanoyl, 2-carboxybenzoyl, nicotinoyl and benzyloxycarbonyl-leucyl-leucyl.

2. The compound according to claim 1, wherein n is 1 and R is 3-carboxypropanoyl.

3. The compound according to claim 1, wherein n is 1 and R is nicotinoyl.

4. The compound according to claim 1, wherein n is 1 and R is benzyloxycarbonyl-leucyl-leucyl.

5. The compound according to claim 1, wherein n is 2 and R is 3-carboxypropanoyl.

6. The compound according to claim 1, wherein n is 2 and R is 2-carboxybenzoyl.

7. The compound according to claim 1, wherein n is 2 and R is nicotinoyl.

8. Process for the preparation of peptide derivatives of the general formula (I) in claim 1 wherein n is an integer of 1 or 2 and R is carboxypropanoyl, 2-carboxybenzoyl or nicotinoyl, characterized by reacting the compound of the formula:

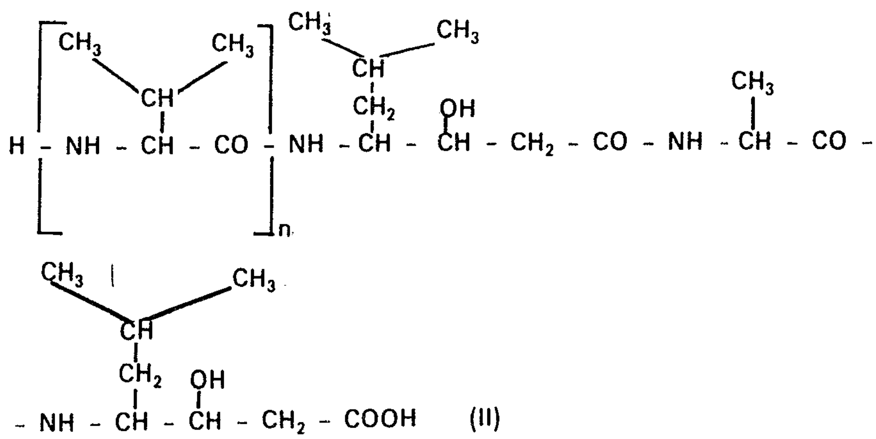

(II)

wherein n is an integer of 1 or 2 with an organic carboxylic acid selected from the group consisting of succinic, and nicotinic acids or reactive derivatives thereof.

9. Process for the preparation of peptide derivatives of the general formula (I) in claim 1, wherein n is an integer of 1 or 2 and R is benzyloxycarbonyl-leucyl-leucyl, characterized by reacting compound of the formula:

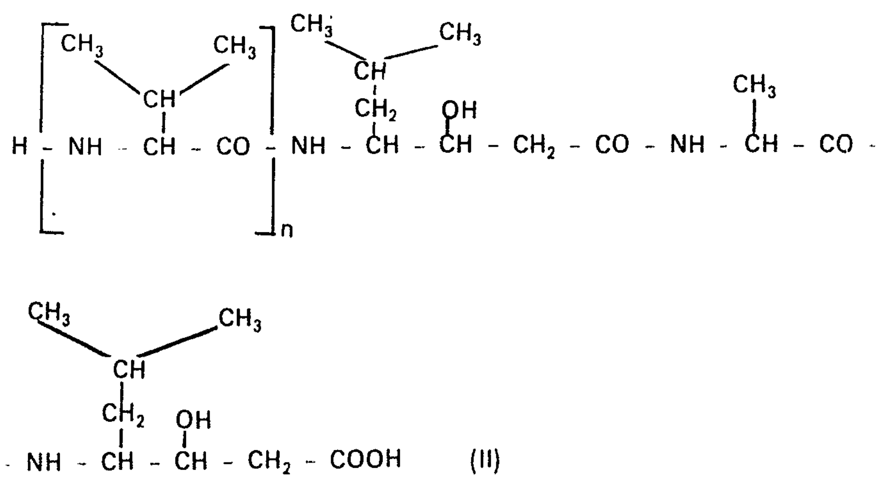

(II)

wherein n is an integer of 1 or 2 with an N-protecting leucine ester, splitting off the N-protecting group of the resulting product, and then reacting the resulting compound with benzyloxy carbonyl-leucine ester.

**Patentansprüche**

1. Peptid-Derivate entsprechend der allgemeinen Formel

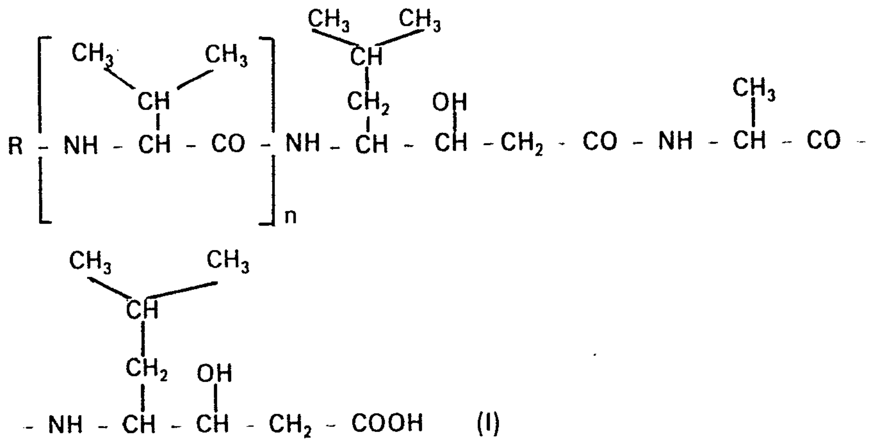

(I)

worin n die ganze Zahl 1 oder 2 bedeutet, und falls n 1 darstellt, R einen Rest der Gruppe Carboxypropanoyl, Nicotinoyl oder Benzyl-oxycarbonyl-leucyl-leucyl darstellt, und wenn n 2 bedeutet, R einen Rest aus der Gruppe Carboxypropanoyl, 2-Carboxybenzoyl, Nicotinoyl und Benzyloxycarbonyl-leucyl-leucyl darstellt.

2. Verbindung gemäss Anspruch 1, dadurch gekennzeichnet, dass n 1 bedeutet und R 3-Carboxypropanoyl darstellt.

3. Verbindung gemäss Anspruch 1, dadurch gekennzeichnet, dass n 1 bedeutet und R Nicotinoyl darstellt.

4. Verbindung gemäss Anspruch 1, dadurch gekennzeichnet, dass n 1 bedeutet und R Benzyloxycarbonyl-leucyl-leucyl darstellt.

5. Verbindung gemäss Anspruch 1, dadurch gekennzeichnet, dass n 2 bedeutet und R 3-Carboxypropanoyl darstellt.

6. Verbindung gemäss Anspruch 1, dadurch gekennzeichnet, dass n 2 bedeutet und R 2-Carboxybenzoyl darstellt.

7. Verbindung gemäss Anspruch 1, dadurch gekennzeichnet, dass n 2 bedeutet und R Nicotinoyl darstellt.

8. Verfahren zur Herstellung von Peptid-Derivaten der allgemeinen Formel (I) in Anspruch 1, wobei n eine ganze Zahl 1 oder 2 darstellt und R Carboxypropanoyl, 2-Carboxybenzoyl oder Nicotinoyl bedeutet, gekennzeichnet durch Umsetzung der Verbindung der Formel

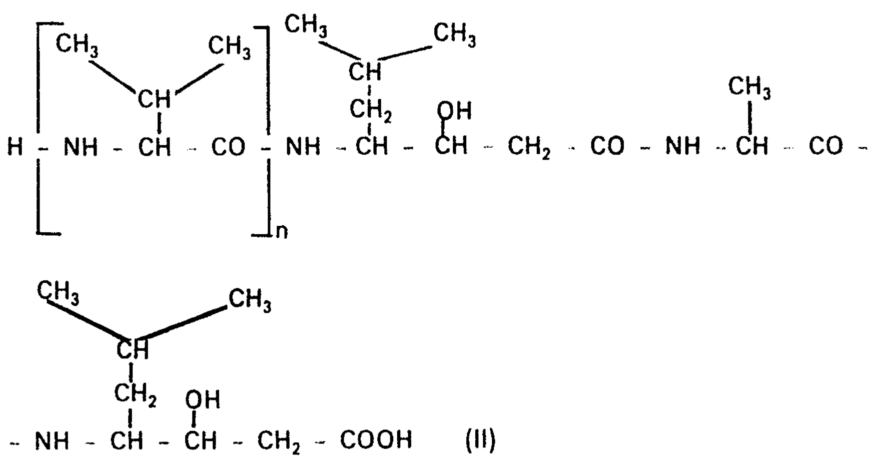

(II)

worin n eine ganze Zahl 1 oder 2 darstellt, mit einer organischen Carbonsäure aus der Gruppe Bernsteinsäure und Nikotinsäuren oder reaktionsfähigen Derivaten derselben.

9. Verfahren zur Herstellung von Peptid-Derivaten der allgemeinen Formel (I) in Anspruch 1, wobei n eine ganze Zahl 1 oder 2 darstellt und R Benzyloxycarbonyl-leucyl-leucyl bedeutet, gekennzeichnet durch Umsetzung der Verbindung der Formel

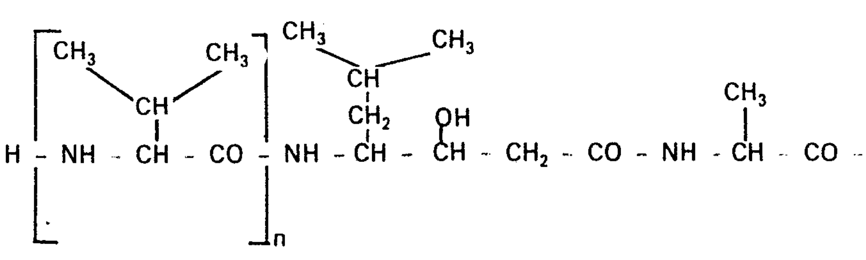

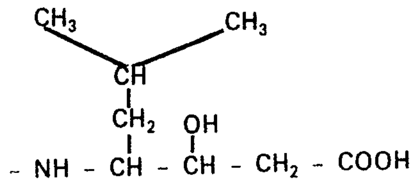

(II)

worin n eine ganze Zahl 1 oder 2 darstellt, mit einem N-geschützten Leucinester, Abspalten der N-Schutzgruppe von dem erhaltenen Produkt, und Umsetzung der erhaltenen Verbindung mit Benzyloxy-carbonyl-leucinester.

**Revendications**

1. Dérivés de peptides représentés par la formule générale:

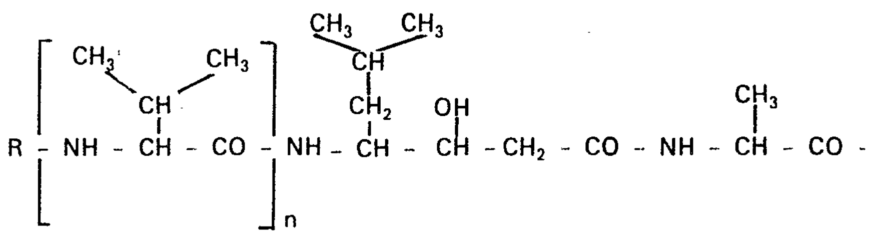

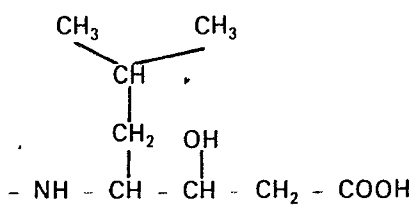

(I)

où n est un nombre entier de 1 ou 2 et, si n est 1, R représente un radical choisi parmi le groupe constitué par carboxypropanoyle, nicotinoyle et benzyloxycarbonyl-leucyl-leucyle, et, si n est 2, R représente un radical choisi parmi le groupe constitué par carboxypropanoyle, carboxy-2 benzoyle, nicotinoyle et benzyloxycarbonyl-leucyl-leucyle.

2. Le composé selon la revendication 1, où n est 1 et R est un carboxy-3 propanoyle.

3. Le composé selon la revendication 1, où n est 1 et R est un nicotinoyle.

4. Le composé selon la revendication 1, où n est 1 et R est un benzyloxycarbonyl-leucyl-leucyle.

5. Le composé selon la revendication 1, où n est 2 et R est un carboxy-3 propanoyle.

6. Le composé selon la revendication 1, où n est 2 et R est un carboxy-2 benzoyle.

7. Le composé selon la revendication 1, où n est 2 et R est un nicotinoyle.

8. Procédé pour la préparation des dérivés de peptides de formule générale (I) de la revendication 1 dans laquelle n est un nombre entier de 1 ou 2 et R est un carboxypropanoyle, un carboxy-2-benzoyle ou un nicotinoyle, caractérisé par la réaction du composé de formule

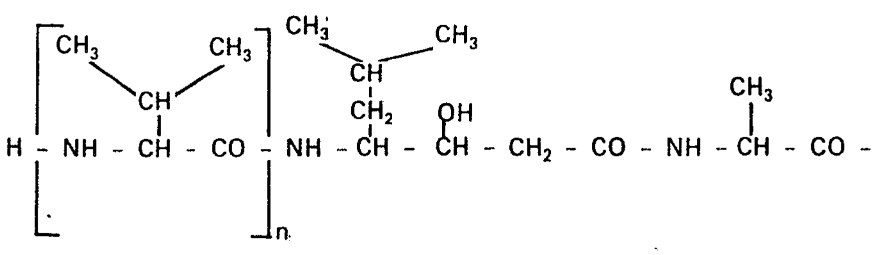

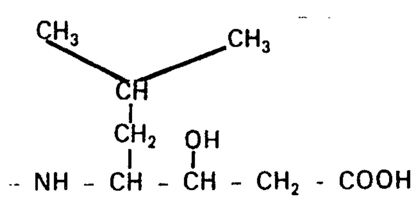

(II)

dans laquelle n est un nombre entier de 1 ou 2, avec un acide carboxylique organique choisi parmi le groupe constitué par les acides succinique et nicotinique ou leurs dérivés réactifs.

9. Procédé pour la préparation des dérivés de peptides de formule générale (I) de la revendication 1 dans laquelle n est un nombre entier de 1 ou 2 et R est un benzyloxycarbonyl-leucyl-leucyle, caractérisé par la réaction du composé de formule:

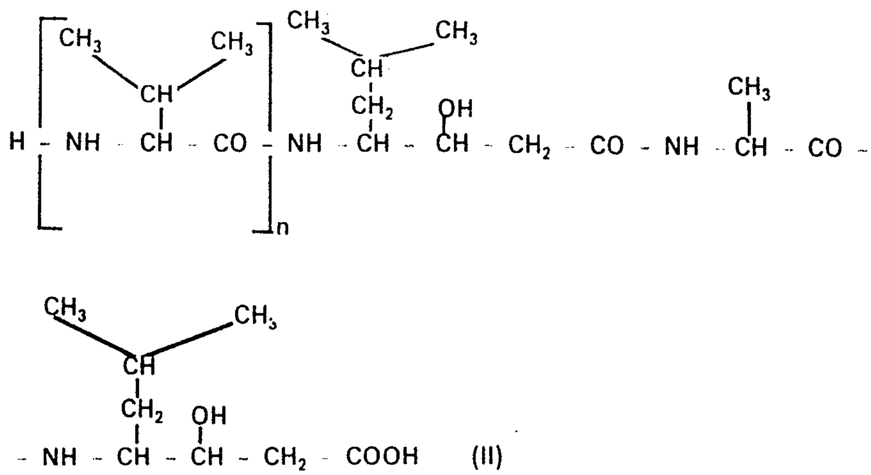

(II)

dans laquelle n est un nombre entier de 1 ou 2, avec un ester de N-protecteur leucine, clivage du groupe N-protecteur du produit obtenu puis réaction du composé obtenu avec un ester de benzyloxycarbonyl-leucine.